# EUROPEAN PATENT APPLICATION

(11) **EP 4 825 377 A1**
(43) Date of publication of application: **21.10.2026**
(21) Application number: 24903887.8
(22) Date of filing: 27.08.2024
(51) Int. Cl.: A61K 31/197, A61P 11/00, A23L 33/10, A61K 9/00

(54) **COMPOSITION FOR IMPROVING RESPIRATORY HEALTH AND TREATING OR PREVENTING RESPIRATORY DISEASES COMPRISING VITAMIN B5, PHARMACEUTICAL COMPOSITION FOR TREATING OR PREVENTING RESPIRATORY DISEASES COMPRISING SAME, AND HEALTH FUNCTIONAL FOOD COMPOSITION FOR IMPROVING RESPIRATORY HEALTH OR RESPIRATORY DISEASES COMPRISING SAME**

(30) Priority: 11.12.2023 KR 20230178581
(71) Applicant: KT&G Corporation, Daedeok-gu Daejeon 34337 (KR)
(72) Inventor: JUNG, Hwa Chul, Daejeon 34128 (KR); LEE, Young Chul, Daejeon 34128 (KR); KIM, Do Hoon, Daejeon 34128 (KR); KIM, So Eun, Daejeon 34128 (KR); MOON, Sung Ok, Daejeon 34128 (KR); SHIN, Han Jae, Daejeon 34128 (KR); LEE, Bo Young, Daejeon 34128 (KR)
(74) Representative: AWA Sweden AB
(86) International application number: PCT/KR2024/012776
(87) International publication number: WO 2025/127316

(57) **Abstract**

The present invention relates to: a composition for improving respiratory health and treating or preventing respiratory diseases, comprising vitamin B5; a pharmaceutical composition for treating or preventing respiratory diseases, comprising same; and a health functional food composition for improving respiratory health or respiratory diseases, comprising same.

## Description

### TECHNICAL FIELD

One or more embodiments relate to a composition including vitamin B₅ for improving respiratory health and treating or preventing respiratory diseases, a pharmaceutical composition including the same for treating or preventing respiratory diseases, and a health functional food composition including the same for improving respiratory health or respiratory diseases.

### BACKGROUND ART

Generally, health functional foods are orally taken in the form of capsules, tablets, etc. However, when the health functional foods are orally taken, there is a problem in that materials are decomposed or excreted through metabolism and decomposition when absorbed into the body.

Meanwhile, vitamin B₅ has various effects such as preventing hair loss, relieving stress, relieving depression, enhancing immunity, and preventing anemia when taken orally. The recommended daily intake for adults is 10 mg, but the daily oral dose required for humans is 100 to 1000 mg, which has a disadvantage of requiring a high dose. There is little research on other methods of administration of vitamin B5 other than oral administration.

### DISCLOSURE OF THE INVENTION

### TECHNICAL GOALS

One or more embodiments provide a composition including vitamin B₅ as an active ingredient, which improves respiratory health even in a small amount, has a respiratory protective effect, and has an effect of improving, preventing, and treating respiratory diseases.

However, goals to be achieved are not limited to those described above, and other goals not mentioned above can be clearly understood by one of ordinary skill in the art from the following description.

### TECHNICAL SOLUTIONS

According to an aspect, there is provided a composition for improving respiratory health and treating or preventing respiratory diseases, including vitamin B₅ as an active ingredient.

According to another aspect, there is provided a pharmaceutical composition for treating or preventing respiratory diseases, including the composition for improving respiratory health and treating or preventing respiratory diseases according to an embodiment.

According to another aspect, there is provided a health functional food composition for improving respiratory health or respiratory diseases, including the composition for improving respiratory health and treating or preventing respiratory diseases according to an embodiment.

### EFFECTS OF THE INVENTION

According to embodiments, when the composition is used, the total number of cells, inflammatory cells, and inflammatory cytokines in a bronchoalveolar lavage fluid may be reduced, thereby improving respiratory health, improving respiratory diseases including respiratory inflammation, allergies, and asthma, and protecting against respiratory injury caused by particulate matter or pathogens. In addition, it is possible to have an effect of improving respiratory health and treating or preventing respiratory diseases even with a smaller amount than when administered orally by using an inhalation method.

It should be understood that the effects of the present disclosure are not limited to the effects described above, but include all effects that can be deduced from the detailed description of the present disclosure or configurations of the disclosure described in claims.

### BRIEF DESCRIPTION OF THE DRAWINGS

FIG. 1 illustrates results of evaluating the total cell count in a bronchoalveolar lavage fluid according to Examples and Comparative Examples of the present disclosure;
FIG. 2 illustrates results of evaluating effects of inhibiting inflammatory cells (neutrophils) in a bronchoalveolar lavage fluid according to Examples and Comparative Examples of the present disclosure;
FIG. 3 illustrates results of confirming the number of neutrophils in a bronchoalveolar lavage fluid using an immunofluorescence staining (FACS) marker according to Examples and Comparative Examples of the present disclosure; and
FIG. 4 illustrates results of confirming a decrease in inflammatory factor cytokine IL-6 in a bronchoalveolar lavage fluid according to Examples and Comparative Examples of the present disclosure.

### MODE FOR CARRYING OUT THE INVENTION

Hereinafter, embodiments will be described in detail with reference to the accompanying drawings. However, since various modifications may be made to embodiments, the scope of the present disclosure is not limited or restricted by these embodiments. It should be understood that all modifications, equivalents and substitutes for embodiments are included in the scope of the present disclosure.

The terms used in the embodiments are used for the purpose of description only, and should not be construed to be limited. A singular expression includes a plural expression unless the context clearly indicates otherwise. In this specification, it should be understood that the term "including" or "having" indicates that a feature, a number, a step, an operation, a component, a part or the combination thereof described in the specification is present, but does not exclude a possibility of presence or addition of one or more other features, numbers, steps, operations, components, parts or combinations, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as an ideal meaning or excessively formal meanings unless clearly defined in the present application.

In addition, in the description with reference to the accompanying drawings, like components designate like reference numerals regardless of reference numerals and a duplicated description thereof will be omitted. In description of the embodiments, a detailed description of related known technologies will be omitted if it is determined that they unnecessarily make the gist of the embodiments unclear.

In describing the components of the embodiments of the present disclosure, terms including first, second, A, B, (a), (b), and the like may be used. These terms are just intended to distinguish the components from other components, and the terms do not limit the nature, sequence, or order of the components.

Components included in any one embodiment and components having a common function will be described using the same names in other embodiments. Unless otherwise stated, descriptions described in any one embodiment may also be applied to other embodiments, and detailed descriptions in the overlapping range will be omitted.

Throughout the specification, when a certain part "comprises" a certain component, it will be understood to imply the inclusion of stated elements but not the exclusion of any other elements.

According to an embodiment, a composition for improving respiratory health and treating or preventing respiratory diseases, including vitamin B₅ (pantothenic acid) as an active ingredient may be provided.

The vitamin B₅ is a water-soluble vitamin belonging to a vitamin B complex, and is an essential substance for generating collagen, which constitutes the skin and hair, and may also help in generating clean skin and healthy hair by decomposing lipids and preventing oily skin. The vitamin B₅ may have an anti-inflammatory effect as well as effects of preventing hair loss, relieving stress, relieving depression, improving immunity, and preventing anemia.

As used herein, the term "improvement" or "treatment" may indicate all actions that improve or beneficially change the symptoms of respiratory health or diseases by administering the composition. In addition, the term "prevention" indicates all actions that inhibit or delay the occurrence, spread or recurrence of respiratory diseases by administering the composition according to an embodiment of the present disclosure.

As used herein, the term "respiratory organ" refers to an organ capable of inhaling oxygen in the air and exhaling carbon dioxide produced as a result of energy metabolism, and may refer to the airway, bronchi, lungs, alveoli, etc. When there is an invasion that causes any fundamental change in the cells or tissues of these respiratory organs, for example, due to bacteria, viruses, smoking, air pollution, etc., inflammation may occur as a defense response process of the living body to repair or regenerate the damaged area. This series of responses involves local blood vessels, various tissue cells in body fluids, immune-related cells, etc.

As used herein, the term "respiratory diseases" refer to negative symptoms that may occur in the respiratory organs. The respiratory diseases are not limited to diseases that are caused by inflammation in the respiratory organs, but may be at least one disease selected from the group consisting of allergies, bronchitis, sinusitis, lower respiratory tract infection, bronchiolitis, asthma, chronic obstructive pulmonary disease, rhinitis, pneumonia, emphysema, bronchiectasis, pulmonary fibrosis, cough, sputum, pharyngitis, tonsillitis, and laryngitis.

In addition, the composition according to an embodiment of the present disclosure may have an improving effect on the deterioration of respiratory health that may be caused by external factors such as particulate matter, pathogens, and viruses. As used herein, the "improving respiratory health" indicates reducing the intensity and frequency of one or more respiratory symptoms selected from the group consisting of swollen tonsils, sore throat, cough, sneezing, phlegm, sore throat, stuffy nose, and hoarseness of voice, eliminating the respiratory symptoms, or preventing the respiratory symptoms.

The composition for improving respiratory health and treating or preventing respiratory diseases according to an embodiment may be intended for all animals, including humans, that have developed or are likely to develop respiratory diseases. These animals may be cattle, horses, sheep, pigs, goats, camels, antelope, dogs, cats, rats, mice, and the like that require treatment for similar symptoms thereto, as well as humans, but are not limited thereto.

The composition for improving respiratory health and treating or preventing respiratory diseases according to an embodiment may be applied to inflammation by inhalation or spraying.

Here, the inhalation or spraying may be applied to the oral or nasal cavity. Desirably, it may be more effective to apply fine droplets directly around the throat in the oral cavity mainly having respiratory inflammation, by inhalation or spraying the composition through the oral cavity. In the present disclosure, the composition may be administered by inhalation or spraying to avoid metabolism (decomposition) in body tissues, and may be absorbed immediately to improve and treat respiratory inflammation with a smaller amount.

The inhalation or spraying may be applied by any one of an ultrasonic device, a spray device, and a misting device. Here, the composition for improving and treating respiratory inflammation may be administered to a user from the device through a method such as heating, ultrasound, or surface waves, but is not limited to the described method. In particular, when administered by the heating method, the delivery efficiency and speed of the composition for improving and treating respiratory inflammation according to an embodiment of the present disclosure may increase.

In the composition for improving respiratory health and treating or preventing respiratory diseases according to an embodiment, the composition for improving respiratory health and treating or preventing respiratory diseases may be formulated and used in an inhalable form according to a conventional method.

For example, the composition for improving respiratory health and treating or preventing respiratory diseases may be in a liquid form or may be sprayable. If the present disclosure is the liquid, the composition may be aerosolized and sprayed through methods such as heating, ultrasound, and surface waves.

In the composition for improving respiratory health and treating or preventing respiratory diseases according to an embodiment, a dose may be in a range of 1 mg/kg to 30 mg/kg.

The daily oral dose of vitamin B₅ for humans may be as high as 50 mg/kg or more, but the present disclosure may have excellent effects of improving and treating respiratory inflammation even in a lower dose through the inhalation method.

The present disclosure may have effects of improving respiratory health and treating or preventing respiratory diseases within the numerical range of the dose described above. Desirably, the dose of the composition for improving respiratory health and treating or preventing respiratory diseases may be in a range of 1 mg/kg to 30 mg/kg, desirably 5 mg/kg to 28 mg/kg, and more desirably 8 mg/kg to 28 mg/kg. If the dose of the composition is less than the numerical range described above, the effects of improving and treating respiratory diseases may be minimal. On the other hand, even if the dose of the composition exceeds the numerical range described above, the effects of improving, treating, and preventing respiratory diseases are not further improved.

The dose of the composition for improving respiratory health and treating or preventing respiratory diseases according to an embodiment may be achieved, for example, by controlling the concentration, administration time, administration frequency, etc. of the composition.

For example, in the composition for improving respiratory health and treating or preventing respiratory diseases according to an embodiment, vitamin B₅ may have a concentration of 2 wt% to 30 wt%, desirably 5 wt% to 25 wt%, and more desirably 10 to 20 wt%, with respect to the total weight of the composition for improving respiratory health and treating or preventing respiratory diseases, and the concentration may be administered for 5 minutes to 60 minutes. However, the method of achieving the dose is not limited to the embodiments described above.

According to an embodiment, a health functional food composition for improving respiratory health or respiratory diseases, including the composition for improving respiratory health and treating or preventing respiratory diseases may be provided.

The health functional food composition refers to food produced and processed using raw materials or ingredients with functionality, which are useful for the human body according to the Art on Health Functional Foods No. 6727, and the "functionality" may include intake for adjusting nutrients for the structures and functions of the human body or obtaining a useful effect on health applications such as physiological actions.

According to an embodiment, a pharmaceutical composition for treating or preventing respiratory diseases, including the composition for improving respiratory health and treating or preventing respiratory diseases may be provided.

The pharmaceutical composition may include a composition manufactured for the purpose of preventing or treating diseases. The composition for improving and treating respiratory inflammation may be used alone or in combination with other pharmaceutically active compounds exhibiting effects of preventing and treating respiratory inflammatory diseases or in an appropriate set thereof.

According to an embodiment, a method of treating or preventing respiratory diseases, including administering to a subject the pharmaceutical composition for treating or preventing respiratory diseases may be provided. The subject may include all animals including the human, and may be humans, cattle, horses, sheep, pigs, goats, camels, antelope, dogs, cats, rats, mice, and the like, but is not limited thereto.

According to an embodiment, a use of the pharmaceutical composition for treating or preventing respiratory diseases for treating or preventing respiratory diseases may be provided.

Hereinafter, the present disclosure will be described in more detail with reference to examples, but the present disclosure is not limited to the following examples.

### 1. Preparation of Inhalation Composition for Improving and Treating Respiratory Inflammation

25, 100, and 200 g of vitamin B₅ (Pantothenic Acid, 0412678, DSM, UK) were measured and put in 1 L beakers, respectively, added with 700 ml of distilled water, and mixed for 15 minutes using a stirrer. The total amount was adjusted to 1,000 ml with primary distilled water and mixed for 15 minutes to finally prepare an inhalation composition for improving and treating respiratory inflammation containing vitamin B₅ at concentrations of 2.5%, 10%, and 20%.

### 2. Preparation of Oral Composition for Improving and Treating Respiratory Inflammation

To orally administer low, medium, and high doses of vitamin B₅ (Pantothenic Acid, 0412678, DSM, UK), 150, 300, and 600 mg of vitamin B₅ were measured and put into 250 ml glass bottles, respectively. 100 ml of primary distilled water was added to each 250 ml glass bottle and mixed for 15 minutes using a stirrer to prepare an oral composition for improving and treating respiratory inflammation. A test substance prepared for each dose was orally administered using a gastric zonde at a dose of 10 ml/kg.

### 3. Preparation of Respiratory Injury Model Administered with Composition for Improving and Treating Respiratory Inflammation

### (1) Experimental animal

As an experimental animal, Balb/c mice (male, 6 weeks old, body weight of 15 to 17 g) were purchased from Coretech after receiving approval from the Institutional Animal Care and Use Committee (IACUC). After acclimatization for one week in the breeding room of the KT&G Bio-Efficacy Evaluation Center (temperature 23 ± 2°C humidity 50 ± 10%, 12-hour light/dark cycle), the mice were used in the experiment.

### (2) Preparation of Test Substance for Inducing Respiratory Injury Model

A PM10D mixture was prepared by diluting 3 mg/mL of particulate matter 10 (hereinafter PM10) [ERM-CZ120, Sigma Aldrich, St. Louis, MO, USA] and 0.6 mg/mL of diesel particulate matter (hereinafter DEP) [NIST-2975, Sigma Aldrich, St. Louis, MO, USA] in 1% Alum (Aluminium Hydroxide, 239186, Sigma Aldrich, USA).

### (3) Fabrication of Respiratory Injury Model

The experimental animals were pre-anesthetized with isoflurane, 50 µl of PM10D was administered three times (3, 6, and 9 days after administration of the test substance) through the nose and mouth, respectively (100 µl in total), to fabricate a respiratory injury inflammation model.

### (4) Preparation of Respiratory Injury Model Administered with Composition for Improving and Treating Respiratory Inflammation

5 to 6 animals were used per group, and as Comparative Examples 1 and 2, normal animals that were not treated at all (normal group) and a respiratory injury inflammation model group (induced group) inhaled with purified water (60 min/day) after PM10D administration were prepared, and oral administration groups of Comparative Examples 3 to 5 were prepared. As Examples 1 to 8, 2.5, 10, and 20% of vitamin B₅ were inhaled for 10 days for each dose (mg/kg) by adjusting the administration time after PM10D treatment, and then inflammation improving effects were compared. The inhalation was performed using a mist generator. The exposure amount of aerosol (mg/L) was calculated as a dose (mg/kg) using a calculation formula based on a prior art (Alexander et al., Inhal Toxicol. 2008 Oct;20(13):1179-89.).

**[Table 1]**

| Classification | Group | Concentration (%) of vitamin B₅ | Administration time (min) |
|---|---|---|---|
| Comparative Example 1 | Normal group | - | - |
| Comparative Example 2 | Induced group | Administer purified water instead of vitamin B₅ | 60 |
| Comparative Example 3 | 15 mg/kg (oral administration group) | - | - |
| Comparative Example 4 | 30 mg/kg (oral administration group) | - | - |
| Comparative Example 5 | 60 mg/kg (oral administration group) | - | - |
| Example 1 | 1 mg/kg | 2.5 | 10 |
| Example 2 | 2 mg/kg | 10 | 7 |
| Example 3 | 6 mg/kg | 10 | 20 |
| Example 4 | 10 mg/kg | 10 | 37 |
| Example 5 | 10 mg/kg | 20 | 22 |
| Example 6 | 12 mg/kg | 20 | 26 |
| Example 7 | 17 mg/kg | 10 | 60 |
| Example 8 | 28 mg/kg | 20 | 60 |

### 4. Experimental Example 1: Evaluation of Total Cell Count in Bronchoalveolar Lavage Fluid

The total cell counts in the bronchoalveolar lavage fluids (BALF) of Examples 1 to 8 and Comparative Examples 1 to 5 were examined. The examination method was as follows.

In Examples 1 to 8 and Comparative Examples 1 to 5, on day 11 after the end of administration, the experimental animals were anesthetized with isoflurane, blood was collected, the airway was exposed, a syringe containing 1 mL of phosphate buffered saline (10010-023, Gibco, UK) was inserted into the trachea, and then fixed by tying with thread. A bronchoalveolar lavage fluid was repeated and obtained three times using phosphate buffered saline. After centrifugation (1500 RPM/4°C/10 min) of the obtained bronchoalveolar lavage fluid, the supernatant was stored in an ultra-low temperature freezer (-70°C) to measure cytokines, and cells isolated from the bronchoalveolar lavage fluid were treated with ammonium-chloride-potassium buffer (ACK, A10492-01, Gibco, NY, USA) solution for 10 minutes to lyse red blood cells, and then washed again with phosphate buffered saline, and the total cell count was measured using a hematocytometer.

The results obtained from the experiments were recorded as the mean ± standard deviation, and statistical processing was performed using one-way ANOVA using GraphPad Prism 9.5 (GraphPad Software, Inc., Lajolla, CA, USA) to determine statistically significant variance, and if the significance was recognized, a multiple analysis comparison method (Dunnett's multiple comparison test) was used. Significance tests were performed at a *P* value *< 0.05 (*), < 0.01 (**), < 0.001 (***),* or *< 0.0001 (****)* or lower.

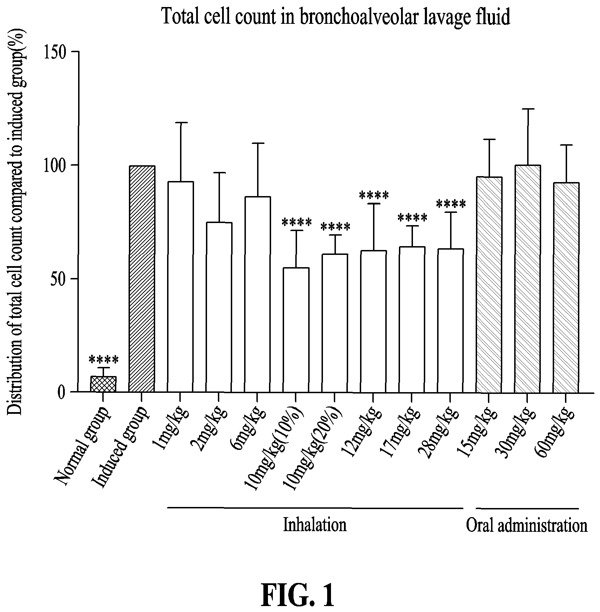

The results are shown in Table 2 below and FIG. 1.

**[Table 2]**

| Classification | Compared to induced group (%) |
|---|---|
| Comparative Example 1 | 7.2 ± 3.5**** |
| Comparative Example 2 | 100 |
| Comparative Example 3 | 95.4 ± 16.3 |
| Comparative Example 4 | 100.9 ± 24.6 |
| Comparative Example 5 | 93.1 ± 16.2 |
| Example 1 | 93.2 ± 25.6 |
| Example 2 | 75.5 ± 21.2 |
| Example 3 | 86.3 ± 23.5 |
| Example 4 | 55.2 ± 16.5**** |
| Example 5 | 61.4 ± 8.2*** |
| Example 6 | 63.1 ± 20.6**** |
| Example 7 | 64.6 ± 9.2**** |
| Example 8 | 63.9 ± 15.8**** |

Referring to Table 2 and FIG. 1, in Examples 2 to 8, the total cell count in the bronchoalveolar lavage fluid was reduced compared to Comparative Examples 2 to 5, and particularly, in Examples 3 to 8, the total cell count in bronchoalveolar lavage fluid was significantly reduced compared to Comparative Example 2 or Comparative Examples 3 to 5. That is, the result was shown that the total cell count in the bronchoalveolar lavage fluid was reduced compared to the induced group and the oral administration group when vitamin B₅ was inhaled at 6 mg/kg or more. Through this, it was confirmed that when vitamin B₅ was administered in a predetermined dose or more, the vitamin B₅ contributed to reducing inflammation levels, and the inflammation level reduction effect was particularly excellent when administered by inhalation.

### 5. Experimental Example 2: Evaluation of Effect of Inhibiting Inflammatory Cells (Neutrophils) in Bronchoalveolar Lavage Fluid

The effects of inhibiting inflammatory cells in the bronchoalveolar lavage fluids of Examples 1 to 8 and Comparative Examples 1 to 5 were evaluated. The evaluation method was as follows.

The bronchoalveolar lavage fluid was smeared on the slide and subjected to Diff-Quik (38721, Sysmex, Japan) staining according to the manufacturer's manual, and 200 monocytes and polynucleated cells were counted using an optical microscope at 400 x magnification. Changes in the number of neutrophils were confirmed through Diff-Quik staining, and the results obtained from the experiment were recorded as the mean ± standard deviation, and a significance test was performed at a *P* value of < *0.05 (*),* < *0.01 (**)*, < *0.001 (***)*, or < *0.0001 (****)* or lower using the same method described above.

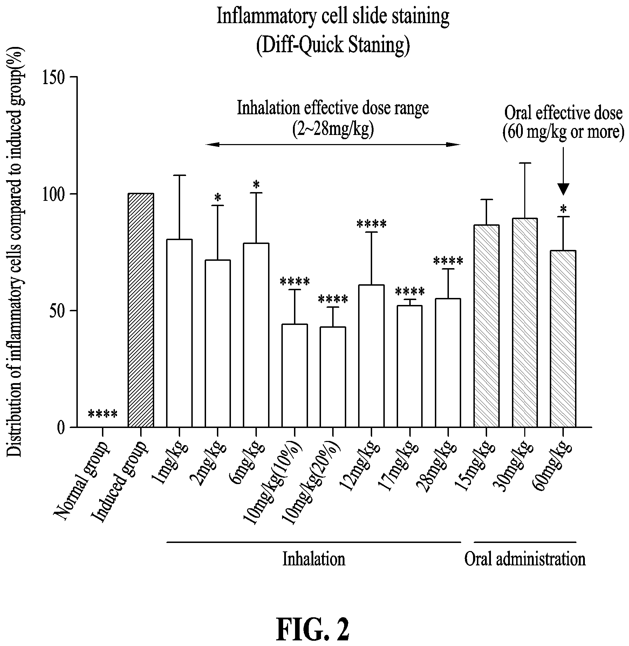

The results are shown in Table 3 and FIG. 2 below.

**[Table 3]**

| Classification | Compared to induced group (%) |
|---|---|
| Comparative Example 1 | 0.1 ± 0.1 **** |
| Comparative Example 2 | 100 |
| Comparative Example 3 | 86.3 ± 11.3 |
| Comparative Example 4 | 89.4 ± 23.5 |
| Comparative Example 5 | 75.8 ± 14.1* |
| Example 1 | 80.1 ± 27.5* |
| Example 2 | 71.4 ± 23.3*** |
| Example 3 | 84.6 ± 24.3* |
| Example 4 | 44.1 ± 14.6**** |
| Example 5 | 43.0 ± 8.2**** |
| Example 6 | 61.0 ± 22.7**** |
| Example 7 | 52.1 ± 2.7**** |
| Example 8 | 54.7 ± 12.9**** |

As can be seen in Table 3 and FIG. 2, in Comparative Example 2, when a particulate matter model was induced in experimental animals with PM10D, the number of neutrophils significantly increased, and thus it was confirmed that the respiratory inflammation level increased by PM10D. In addition, it was confirmed that the number of neutrophils decreased only when a high dose of 60 mg/kg or more was administered orally.

However, when vitamin B₅ was inhaled in Examples 1 to 8, it was confirmed that the number of neutrophils was decreased compared to Comparative Example 2. A significant reduction in the number of neutrophils was observed in Examples 1 to 8 inhaled compared to Comparative Examples 3 and 4. Only when administered at a high dose of 60 mg/kg or more, similarly to the inhalation, it was confirmed that the number of neutrophils was decreased, and thus it was confirmed that the composition according to an embodiment of the present disclosure may secure excellent effects even at a low dose. In particular, it was confirmed that the number of neutrophils was significantly decreased in Examples 3 to 8.

### 6. Experimental Example 3: Confirmation of Effects Using Immunofluorescent Staining Marker in Bronchoalveolar Lavage Fluid

The number of neutrophils (CD11b + Gr-1 + Siglec-F-) in the bronchoalveolar lavage fluid of Examples 2 to 6 and 8 and Comparative Examples 1 to 5 was examined using an immunofluorescent staining marker. The examination method was as follows.

For flow cytometry, cells obtained from the bronchoalveolar lavage fluid were washed in an FACS buffer (1.5% FBS in PBS), and fluorescent substances capable of staining live/dead cells and antibodies for FACS recognizing cell surface markers were diluted in the FACS buffer and attached at 4°C for 20 minutes. Information on the fluorescent substances and antibodies used is provided in Table 4 below.

**[Table 4]**

| Product name | Dilution ratio | Manufacturer | Cat. No |
|---|---|---|---|
| FITC anti-mouse CD4 Antibody (GK1.5) | 1 : 300 | BioLegend | 100405 |
| PerCP/Cy5.5 anti-mouse CD8a Antibody (53-6.7) | 1 : 300 | BioLegend | 100733 |
| PE anti-mouse/human CD44 Antibody (IM7) | 1 : 300 | BioLegend | 103023 |
| PE/Cyanine7 anti-mouse CD62L Antibody (MEL-14) | 1 : 300 | BioLegend | 104417 |
| APC anti-mouse CD69 Antibody (H1.2F3) | 1 : 300 | BioLegend | 104513 |
| Alexa Fluor^{®} 488 Rat Anti-Mouse Siglec-F (E50-2440) | 1 : 300 | BD Biosciences | 567005 |
| PE anti-mouse Ly-6G/Ly-6C (Gr-1) Antibody (RB6-8C5) | 1 : 300 | BioLegend | 108407 |
| APC anti-mouse/human CD11b Antibody (M1/70) | 1 : 300 | BioLegend | 101211 |
| Ghost Dye^{™} Red 780 Fixable Viability Dye | 1 : 1000 | Cell Signaling Technology | 18452S |

After incubation, the cells were washed with the FACS buffer and fixed with a fixation buffer (1% PFA, 4% PFA solution diluted 1/4 in PBS). Samples were acquired and fluorescence signals were detected using an FACS CantoII (BD Biosciences, San Diego, CA, USA) instrument and analyzed using FlowJo (Treestar) software. The number of CD11b + Gr-1 + Siglec-F- cells was measured, and the results obtained from the experiment were recorded as the mean ± standard deviation, and a significance test was performed at a *P* value level of *< 0.05 (*)* or *< 0.01 (**)* or lower using the same method described above.

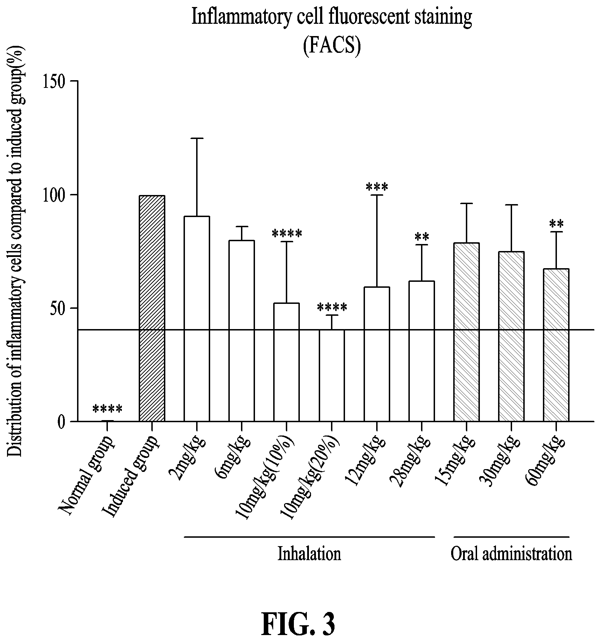

The results are shown in Table 5 and FIG. 3 below.

**[Table 5]**

| Classification | Compared to induced group (%) |
|---|---|
| Comparative Example 1 | 0.2 ± 0.3**** |
| Comparative Example 2 | 100 |
| Comparative Example 3 | 78.8 ± 17.6 |
| Comparative Example 4 | 75.2 ± 20.4* |
| Comparative Example 5 | 67.6 ± 16.4** |
| Example 2 | 90.4 ± 34.3 |
| Example 3 | 80.1 ± 5.9 |
| Example 4 | 52.1 ± 27.4**** |
| Example 5 | 40.5 ± 6.6**** |
| Example 6 | 59.5 ± 40.7*** |
| Example 8 | 62.0 ± 15.8** |

As can be seen in Table 5 and FIG. 3, Example 3 showed a result inhibited by about 20% compared to Comparative Example 2, and in particular, from Example 4, results inhibited by 40 to 60% were shown. In addition, better inhibition results were confirmed in Examples 4 to 6, which were administered at low doses for 20 minutes to 45 minutes, compared to Example 8, which was administered at a high dose for a long period of time.

### 7. Experimental Example 4: Evaluation of Effect on Expression of Inflammatory Factor (IL-6)

The production of cytokine IL-6 was measured in the bronchoalveolar lavage fluids isolated from Examples 2 to 6 and 8 and Comparative Examples 1 to 5 by enzyme-linked immunosorbent assay (ELISA). The examination method was as follows.

To measure the amount of IL-6 (M6000B, R&D systems, Minneapolis, USA) in the bronchoalveolar lavage fluid, an ELISA kit was used. The bronchoalveolar lavage fluid obtained from the experimental animals was stored in a deep freezer and then taken out and measured according to the manufacturer's protocol. After measuring the absorbance at 450 nm, a 540 nm measurement value was corrected and used, and the measurement was performed using SpectraMax i3x (Molecular devices, San Jose, CA, USA) equipment.

The results obtained from the experiment were recorded as the mean ± standard deviation, and a significance test was performed at a *P* value level of *< 0.05 (*)* or *< 0.01 (**)* or lower using the same method described above.

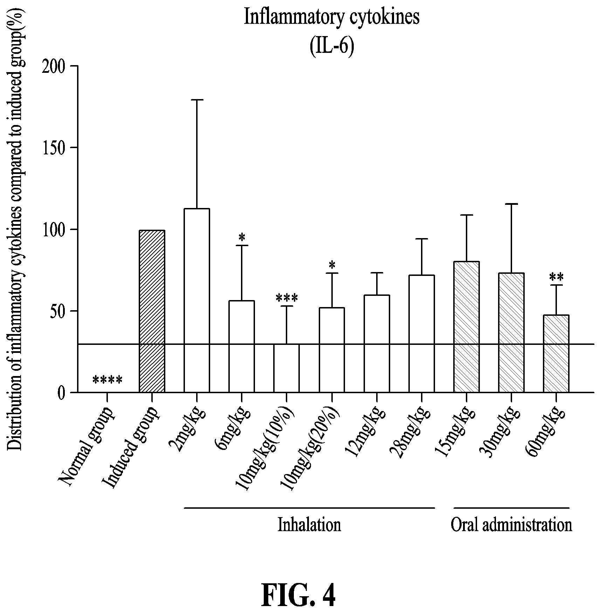

The results are shown in Table 6 and FIG. 4 below.

**[Table 6]**

| Classification | Compared to induced group (%) |
|---|---|
| Comparative Example 1 | 0.1 ± 0.4**** |
| Comparative Example 2 | 100 |
| Example 2 | 112.8 ± 66.9 |
| Example 3 | 56.3 ± 34.1* |
| Example 4 | 29.4 ± 21.3*** |
| Example 5 | 52.5 ± 20.7* |
| Example 6 | 59.9 ± 13.7 |
| Example 8 | 72.0 ± 22.5 |

As can be seen in Table 6 and FIG. 4, it was confirmed that in Comparative Example 2 induced by PM10D, the amount of IL-6, an inflammation-related indicator, was increased compared to Comparative Example 1. However, in Examples 3 to 8 administered with 6 to 28 mg/kg of vitamin B₅, the amount of IL-6 tended to decrease, and the tendency in which the amount of IL-6 was decreased was also confirmed compared to Comparative Examples 3 to 5, which were oral administration groups. In particular, Example 4 administered with 10% vitamin B₅ of 10 mg/kg showed a clear decrease of 70% or more. In addition, better reduction results were confirmed in Examples 3 to 6, which were administered at low doses for 20 minutes to 45 minutes, compared to Example 8, which was administered at a high dose for a long period of time.

From Experimental Examples 1 to 4, it is predicted that by using the composition for improving respiratory health and treating or preventing respiratory diseases described in the appended claims, it is possible to provide a composition having an excellent effect of improving and treating respiratory inflammation even with a small dosage.

While the embodiments are described, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if components in a described system, architecture, device, or circuit are combined in a different manner, or replaced or supplemented by other components or their equivalents.

Therefore, other implementations, other embodiments, and equivalents to the claims are also within the scope of the following claims.

## Claims

1. A composition for improving respiratory health and treating or preventing respiratory diseases, the composition comprising vitamin B₅ as an active ingredient.

2. The composition of claim 1, wherein the composition is applied to inflammation by inhalation or spraying.

3. The composition of claim 2, wherein the inhalation or spraying is applied to an oral or nasal cavity.

4. The composition of claim 2, wherein the inhalation or spraying is applied by one of an ultrasonic device, a spray device, and a misting device.

5. The composition of claim 1, wherein the composition is a liquid.

6. The composition of claim 1, wherein a dose of the composition is in a range of 1 mg/kg to 30 mg/kg.

7. The composition of claim 1, wherein the vitamin B₅ has a concentration of 2 wt% to 30 wt% with respect to a total weight of the composition.

8. The composition of claim 1, wherein the composition is administered for 5 minutes to 60 minutes.

9. The composition of claim 1, wherein the respiratory diseases comprise at least one selected from the group consisting of allergy, bronchitis, sinusitis, lower respiratory tract infection, bronchiolitis, asthma, chronic obstructive pulmonary disease, rhinitis, pneumonia, emphysema, bronchiectasis, pulmonary fibrosis, cough, sputum, pharyngitis, tonsillitis, and laryngitis.

10. A pharmaceutical composition for treating or preventing respiratory diseases, the pharmaceutical composition comprising the composition according to any one of claims 1 to 9.

11. A health functional food composition for improving respiratory health or respiratory diseases, the health functional food comprising the composition according to any one of claims 1 to 9.
